(19) Europäisches Patentamt
European Patent Office
Office européen des brevets

(11) **EP 3 460 050 B1**

(12) **EUROPEAN PATENT SPECIFICATION**

(45) Date of publication and mention
of the grant of the patent:
**03.11.2021 Bulletin 2021/44**

(51) Int Cl.:
*C12N 5/071* (2010.01)        *C12N 5/077* (2010.01)
*C07K 14/435* (2006.01)        *C12N 15/09* (2006.01)
*C12N 5/00* (2006.01)

(21) Application number: **17799463.9**

(22) Date of filing: **18.05.2017**

(86) International application number:
**PCT/JP2017/018657**

(87) International publication number:
**WO 2017/200039 (23.11.2017 Gazette 2017/47)**

(54) **CELL CULTURING METHOD, CULTURE MEDIUM, AND CULTURE MEDIUM KIT**

ZELLZÜCHTUNGSVERFAHREN, KULTURMEDIUM UND KULTURMEDIUMKIT

PROCÉDÉ DE CULTURE CELLULAIRE, MILIEU DE CULTURE ET KIT DE MILIEU DE CULTURE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **19.05.2016 JP 2016100318**

(43) Date of publication of application:
**27.03.2019 Bulletin 2019/13**

(73) Proprietor: **FUJIFILM Corporation**
**Tokyo 106-8620 (JP)**

(72) Inventors:
• **MURAYA, Koji**
**Ashigarakami-gun**
**Kanagawa 258-8577 (JP)**
• **TSUZUKI, Hirohiko**
**Ashigarakami-gun**
**Kanagawa 258-8577 (JP)**
• **AKUTSU, Hidenori**
**Tokyo 157-8535 (JP)**

(74) Representative: **Hoffmann Eitle**
**Patent- und Rechtsanwälte PartmbB**
**Arabellastraße 30**
**81925 München (DE)**

(56) References cited:
WO-A1-2015/111686    WO-A1-2017/082220
WO-A1-2017/117333    JP-A- S60 196 186
JP-A- 2010 519 252    US-A1- 2010 129 910

• **SEZIN ADAY ET AL: "A cost-effective and simple culture method for primary hepatocytes", ANIMAL CELLS AND SYSTEMS, vol. 15, no. 1, 1 March 2011 (2011-03-01), pages 19-27, XP055556205, ISSN: 1976-8354, DOI: 10.1080/19768354.2011.555140**
• **ZHAOLIE C ET AL: "A NOVEL SERUM-FREE MEDIUM FOR THE CULTIVATION OF VERO CELLS ON MICROCARRIERS", BIOTECHNOLOGY TECHNIQUES, CHAPMAN & HALL, vol. 10, no. 6, 1 June 1996 (1996-06-01), pages 449-452, XP000900996, ISSN: 0951-208X, DOI: 10.1007/BF00174231**
• **PETER E HALL ET AL: "Laminin enhances the growth of human neural stem cells in defined culture media", BMC NEUROSCIENCE, vol. 9, no. 1, 1 January 2008 (2008-01-01) , page 71, XP055557338, GB ISSN: 1471-2202, DOI: 10.1186/1471-2202-9-71**
• **NOBUYUKI TAKASU ET AL: "Effect of gelatin on the cyclic AMP response of primocultured hog thyroid cells to acute thyrotropin stimulation", BIOCHIMICA ET BIOPHYSICA ACTA (BBA) - GENERAL SUBJECTS, ELSEVIER, AMSTERDAM, NL, vol. 587, no. 4, 1 November 1979 (1979-11-01), pages 507-514, XP025792335, ISSN: 0304-4165, DOI: 10.1016/0304-4165(79)90004-7 [retrieved on 1979-11-01]**

- **RONAN JAMBOU ET AL: "In vitro culture of Plasmodium berghei-ANKA maintains infectivity of mouse erythrocytes inducing cerebral malaria", MALARIA JOURNAL, BIOMED CENTRAL, LONDON, GB, vol. 10, no. 1, 25 November 2011 (2011-11-25), page 346, XP021112028, ISSN: 1475-2875, DOI: 10.1186/1475-2875-10-346**

SEQUENCE LISTING
<110> FUJIFILM Corporation
<120> A cell culture method, medium and medium kit
<130>\201@17F00470
<160> 10
<170> PatentIn version 3.5
<210> 1
<211> 571
<212> PRT
<213> Recombinant
<400> 1

```
Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
            20                  25                  30
Arg Gly Asp Ala Gly Pro Lys Gly Ala Asp Gly Ala Pro Gly Ala Pro
        35                  40                  45
Gly Leu Gln Gly Met Pro Gly Glu Arg Gly Ala Ala Gly Leu Pro Gly
    50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
            100                 105                 110
Asp Ala Gly Pro Lys Gly Ala Asp Gly Ala Pro Gly Lys Asp Gly Val
        115                 120                 125
Arg Gly Leu Ala Gly Pro Ile Gly Pro Pro Gly Pro Ala Gly Ala Pro
    130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
            165                 170                 175
Asp Gly Ala Pro Gly Lys Asp Gly Val Arg Gly Leu Ala Gly Pro Pro
        180                 185                 190
Gly Ala Pro Gly Leu Gln Gly Ala Pro Gly Leu Gln Gly Met Pro Gly
        195                 200                 205
Glu Arg Gly Ala Ala Gly Leu Pro Gly Pro Lys Gly Glu Arg Gly Asp
    210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
            245                 250                 255
Glu Arg Gly Asp Ala Gly Pro Lys Gly Ala Asp Gly Ala Pro Gly Lys
            260                 265                 270
Asp Gly Val Arg Gly Leu Ala Gly Pro Ile Gly Pro Pro Gly Glu Arg
        275                 280                 285
Gly Ala Ala Gly Leu Pro Gly Pro Lys Gly Glu Arg Gly Asp Ala Gly
    290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
            325                 330                 335
Gly Leu Gln Gly Met Pro Gly Glu Arg Gly Ala Ala Gly Leu Pro Gly
        340                 345                 350
Pro Lys Gly Glu Arg Gly Asp Ala Gly Pro Lys Gly Ala Asp Gly Ala
        355                 360                 365
Pro Gly Lys Asp Gly Val Arg Gly Leu Ala Gly Pro Pro Gly Ala Pro
    370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro
            405                 410                 415
```

20

```
Lys Gly Ala Asp Gly Ala Pro Gly Ala Pro Gly Leu Gln Gly Met Pro
        420                 425                 430
Gly Glu Arg Gly Ala Ala Gly Leu Pro Gly Pro Lys Gly Glu Arg Gly
        435                 440                 445
Asp Ala Gly Pro Lys Gly Ala Asp Gly Ala Pro Gly Lys Asp Gly Val
        450                 455                 460
Arg Gly Leu Ala Gly Pro Ile Gly Pro Pro Gly Glu Arg Gly Ala Ala
465                 470                 475                 480
Gly Leu Pro Gly Pro Lys Gly Glu Arg Gly Asp Ala Gly Pro Lys Gly
                485                 490                 495
Ala Asp Gly Ala Pro Gly Lys Asp Gly Val Arg Gly Leu Ala Gly Pro
                500                 505                 510
Ile Gly Pro Pro Gly Pro Ala Gly Ala Pro Gly Ala Pro Gly Leu Gln
        515                 520                 525
Gly Met Pro Gly Glu Arg Gly Ala Ala Gly Leu Pro Gly Pro Lys Gly
        530                 535                 540
Glu Arg Gly Asp Ala Gly Pro Lys Gly Ala Asp Gly Ala Pro Gly Lys
545                 550                 555                 560
Asp Gly Val Arg Gly Leu Ala Gly Pro Pro Gly
                565                 570
```

<210> 2
<211> 4
<212> PRT
<213> Artificial Sequence
<220>
<223> Description of Artificial Sequence: adhesive sequence
<400> 2

```
Arg Glu Asp Val
1
```

<210> 3
<211> 5
<212> PRT
<213> Artificial Sequence
<220>
<223> Description of Artificial Sequence: adhesive sequence
<400> 3

```
Tyr Ile Gly Ser Arg
1               5
```

<210> 4
<211> 5
<212> PRT
<213> Artificial Sequence
<220>
<223> Description of Artificial Sequence: adhesive sequence
<400> 4

```
Pro Asp Ser Gly Arg
1               5
```

<210> 5
<211> 7
<212> PRT
<213> Artificial Sequence
<220>

<223> Description of Artificial Sequence: adhesive sequence
<400> 5

```
                        Arg Tyr Val Val Leu Pro Arg
                        1               5
```

<210> 6
<211> 6
<212> PRT
<213> Artificial Sequence
<220>
<223> Description of Artificial Sequence: adhesive sequence
<400> 6

```
                        Leu Gly Thr Ile Pro Gly
                        1               5
```

<210> 7
<211> 10
<212> PRT
<213> Artificial Sequence
<220>
<223> Description of Artificial Sequence: adhesive sequence
<400> 7

```
                 Arg Asn Ile Ala Glu Ile Ile Lys Asp Ile
                 1               5                   10
```

<210> 8
<211> 5
<212> PRT
<213> Artificial Sequence
<220>
<223> Description of Artificial Sequence: adhesive sequence
<400> 8

```
                        Ile Lys Val Ala Val
                        1               5
```

<210> 9
<211> 4
<212> PRT
<213> Artificial Sequence
<220>
<223> Description of Artificial Sequence: adhesive sequence
<400> 9

```
                        Asp Gly Glu Ala
                        1
```

<210> 10
<211> 4
<212> PRT
<213> Artificial Sequence
<220>
<223> Description of Artificial Sequence: adhesive sequence
<400> 10

```
Glu Arg Gly Asp
1
```

**Description**

Field of the Invention

The present invention relates to a cell culture method using a medium in which a human-type recombinant protein is dissolved as defined in the appended claims.

Description of the Related Art

In order to culture cells that proliferate in an adhering manner, it is general to culture cells on a culture substrate coated with an extracellular matrix such as fibronectin or collagen. For example, Patent Literature 1 discloses a method for culturing gingival epithelial cells using an incubator coated with an extracellular matrix in culturing gingival epithelial cells, and examples of the extracellular matrix include laminin, collagen, and fibronectin.

It is also known to culture cells by using a medium including a recombinant protein. Patent Literature 2 discloses a method of incubating a mixture of a macromolecular block having biocompatibility and a cell-containing culture solution so as to manufacture a cell structure in which biocompatible macromolecular blocks and cells are included and a plurality of macromolecular blocks are arranged in gaps between a plurality of cells. Patent Literature 3 discloses a culture solution for in vitro fertilization and culture of human embryos in which recombinant human albumin is added in an amount of 0.1 to 2 mass%. Patent Literature 4 discloses a method for culturing human pluripotent stem cells while an undifferentiated state is maintained, the method includes the following steps (a) to (c), in which the (b) and (c) steps are sequentially repeated after the (a) step: (a) culturing human pluripotent stem cells in a first medium which is a medium for pluripotent stem cells including activin; (b) exchanging the first medium with a second medium which is a medium for pluripotent stem cells not including activin so as to culture human pluripotent stem cells; and (c) subculturing human pluripotent stem cells in the first medium.

Aday S et al. describe a cost-effective and simple culture method for primary hepatocytes based on plates coated with collagen, chitosan or gelatin (Aday, Sezin, Nesrin Hasirci, and Ismet Deliloglu Gurhan. "A cost-effective and simple culture method for primary hepatocytes." Animal cells and systems 15.1 (2011): 19-27).

Zhaolie C et al. describe a novel serum-free medium for the cultivation of Vero cells on microcarriers and state that further addition of gelatin and biotin led to the enhanced cell adhesion and spreading without growth promoting activity (Zhaolie, Chen, et al. "A novel serum-free medium for the cultivation of vero cells on microcarriers." Biotechnology techniques 10.6 (1996): 449-452).

Hall PE et al. describe that laminin enhances the growth of human neural stem cells in defined culture media (Hall, Peter E., Justin D. Lathia, and Maeve A. Caldwell. "Laminin enhances the growth of human neural stem cells in defined culture media." BMC neuroscience 9.1 (2008): 71.)

Takasu N et al. describe an effect of gelatin on the cyclic AMP response of primocultured hog thyroid cells to acute thyrotropin stimulation and suggest that these effects of gelatin might be mediated by interaction of the denatured collagen molecules with external proteins of the plasma membrane of thyroid cells (Takasu, Nobuyuki, et al. "Effect of gelatin on the cyclic AMP response of primocultured hog thyroid cells to acute thyrotropin stimulation." Biochimica et Biophysica Acta (BBA)-General Subjects 587.4 (1979): 507-514.)

Jambou R et al. describe that *in vitro* culture of *Plasmodium berghei-ANKA* maintains infectivity of mouse erythrocytes inducing cerebral malaria. Specifically, high quality culture media were used and reinvasion rates were improved by vigorous orbital shaking of the flask and increasing density of the medium with gelatin (Jambou, Ronan, et al. "In vitro culture of Plasmodium berghei-ANKA maintains infectivity of mouse erythrocytes inducing cerebral malaria." Malaria Journal 10.1 (2011): 346.)

WO 2017/117333 A1 describes methods of producing three-dimensional (3-D) microtissue from a starting cell suspension of pluripotent stem cell (PSC)-derived hepatocytes.

JP 2010/519252 A relates to the field of recombinantly produced gelatin and describes that heterogeneously distributed RGD motifs and is particularly useful for several applications involving cell attachment

Prior Art Literatures

Patent Literatures

Patent Literature 1: JP2011-078326A

Patent Literature 2:WO2011/108517A

Patent Literature 3: WO20091122541A

Patent Literature 4: JP2012-175962A

**SUMMARY OF THE INVENTION**

In a case where cells were cultured by using a culture substrate coated with an extracellular matrix, the experimenter was able to coat the culture substrate with the extracellular matrix or a commercially available culture substrate coated with an extracellular matrix was purchased to be used. In a case where the experimenter coats the culture substrate the extracellular matrix, there is a problem in that the number of working processes increases, and in a case where a commercial product is purchased, there is a problem in that types of available substrates are limited. Therefore, there were restrictions in terms of process and cost in mass culture of cells.

An object of the present invention is to provide a cell culture method having excellent cell proliferation ability by a simple process. Another object of the present invention is to provide a medium and a medium kit used in the cell culture method of the embodiment of the present invention as described above.

The present inventors diligently conducted research to solve the above problems and found that, in a cell culture method using a medium in which a human-type recombinant protein is dissolved, it is possible to provide a cell culture method having excellent cell proliferation ability by a simple process by using laminin, collagen, gelatin, or a variant thereof as a human-type recombinant protein and causing the content of a human-type recombinant protein in the medium to 0.01 ng/mL to 500 $\mu$g/mL. The present invention has been completed based on the finding.

The present invention is defined in the appended claims.

Also disclosed is the following.

[1] A cell culture method comprising:

culturing cells in a medium in which a human-type recombinant protein is dissolved,
in which the human-type recombinant protein is laminin, collagen, gelatin, or a variant thereof, and a content of the human-type recombinant protein in the medium is 0.01 ng/mL to 500 $\mu$g/mL.

[2] The cell culture method according to [1], in which a content of the human-type recombinant protein in the medium is 0.01 ng/mL to 300 $\mu$g/mL.

[3] The cell culture method according to [1] or [2], in which the human-type recombinant protein includes recombinant gelatin having an amino acid sequence derived from a partial amino acid sequence of collagen.

[4] The cell culture method according to any one of [1] to [3], in which the human-type recombinant protein has a repeating sequence represented by Gly-X-Y characteristic to collagen, X and Y each independently represent any one of amino acid, a plurality of pieces of the Gly-X-Y may be the same as or different from each other, and a molecular weight of the human-type recombinant protein is 2 kDa to 100 kDa.

[5] The cell culture method according to any one of [1] to [4], in which the human-type recombinant protein has a repeating sequence represented by Gly-X-Y characteristic to collagen, X and Y each independently represent any one of amino acid, a plurality of pieces of the Gly-X-Y may be the same as or different from each other, and a molecular weight of the human-type recombinant protein is 10 kDa to 90 kDa.

[6] The cell culture method according to any one of [1] to [4], in which the human-type recombinant protein has a repeating sequence represented by Gly-X-Y characteristic to collagen, X and Y each independently represent any one of amino acid, a plurality of pieces of the Gly-X-Y may be the same as or different from each other, and the human-type recombinant protein includes two or more sequences of cell adhesion signals in one molecule.

[7] The cell culture method according to [6], in which the cell adhesion signal is an amino acid sequence represented by Arg-Gly-Asp.

[8] The cell culture method according to any one of [1] to [7], in which an amino acid sequence of the human-type recombinant protein is represented by the following formula,

A-[(Gly-X-Y)$_n$]$_m$-B

in the formula, A represents any amino acid or an amino acid sequence, B represents any amino acid or an amino acid sequence, n pieces of X each independently represent any amino acid, n pieces of Y each independently represent any amino acid, and n represents an integer of 3 to 100, m represents an integer of 2 to 10, and n pieces of Gly-X-Y may be the same as or different from each other.

[9] The cell culture method according to any one of [1] to [8], in which an amino acid sequence of the human-type recombinant protein is represented by the following formula,

$$\text{Gly-Ala-Pro-}[(\text{Gly-X-Y})_{63}]_3\text{-Gly}$$

In the formula, 63 pieces of X each independently represent any amino acid and 63 pieces of Y each independently represent any amino acid. 63 pieces of Gly-X-Y may be the same as or different from each other.

[10] The cell culture method according to any one of [1] to [9], in which the human-type recombinant protein has (1) an amino acid sequence described in SEQ ID No: 1, or (2) an amino acid sequence having 80% or more sequence identity to the amino acid sequence described in SEQ ID No: 1 and has cell adhesiveness.

[11] The cell culture method according to any one of [1] to [10], in which the cell is an adherent cell.

[12] A medium comprising: a basal medium component and a dissolved human-type recombinant protein, in which the human-type recombinant protein is laminin, collagen, gelatin, or a variant thereof, and the content of the human-type recombinant protein in the medium is 0.01 ng/mL to 500 μg/mL.

[13] The medium according to [12], further comprising: 5 volume% or less of serum.

[14] A medium kit comprising: a basal medium component and a human-type recombinant protein in a separated manner, in which the human-type recombinant protein is laminin, collagen, gelatin, or a variant thereof, and the content of the human-type recombinant protein in the medium manufactured by mixing the basal medium component and the human-type recombinant protein is 0.01 ng/mL to 500 μg/mL.

The cell culture method of the embodiment of the present invention as defined in the appended claims is a simple process and has excellent cell proliferation ability. According to the medium and the medium kit of the disclosure, it is possible to culture cells having excellent cell proliferation ability by a simple process.

## BRIEF DESCRIPTION OF THE DRAWINGS

Fig. 1 illustrates a cell proliferation curve at passage 5 by using cartilage-derived cells Yub2478.

Fig. 2 illustrates a cell proliferation rate during short-term culture using the cartilage-derived cells Yub2478.

Fig. 3 illustrates a culture vessel.

Fig. 4 illustrates images of cells in a test for examining cell adhesion ability to a surface of a culture bag using bone marrow-derived cells BMSC.

Fig. 5 illustrates a cell adhesion rate to a surface of a culture bag using bone marrow-derived cells BMSC.

Fig. 6 illustrates concentration dependence of the number of cells in a case of adding CBE3.

Fig. 7 illustrates cell proliferation curves at passage 5 using bone marrow-derived cells UDE BM.

Fig. 8 illustrates cell proliferation during short-term culture using the cartilage-derived cells Yub2478.

Fig. 9 illustrates gene expression profiles of extracellular matrices and cell adhesion-related gene groups.

Fig. 10 illustrates gene expression profiles of the extracellular matrices and cell adhesion-related gene groups.

Fig. 11 illustrates gene expression profiles of cell cycle-related gene groups.

Fig. 12 illustrates gene expression profiles of the cell cycle-related gene groups.

## DESCRIPTION OF THE PREFERRED EMBODIMENTS

Hereinafter, an embodiment of the present invention will be described in detail. The cell culture method of the present disclosure is a cell culture method including culturing cells in a medium in which a human-type recombinant protein is dissolved, in which the human-type recombinant protein is laminin, collagen, gelatin, or a variant thereof, and a content of the human-type recombinant protein in the medium is 0.01 ng/mL to 500 μg/mL.

Methods of culturing cells by using a medium including a recombinant protein are disclosed in WO2011/108517A, WO2009/122541A, and JP2012-175962A. However, the method disclosed in WO2011/108517A is a method of manufacturing a cell structure of macromolecular blocks and cells by incubating a mixture of macromolecular blocks having biocompatibility and a cell containing culture solution, and is different from the method of the embodiment of the present invention as defined in the appended claims in which a recombinant protein is dissolved in a medium. WO2009/122541A discloses a culture solution for in vitro fertilization and culture of human embryos including recombinant human albumin, but does not disclose the human-type recombinant protein used in the present invention as defined in the appended claims, and the addition amount of the recombinant human albumin is different from the content of the human-type recombinant protein of the embodiment of the present invention as defined in the appended claims. JP2012-175962A discloses a medium for pluripotent stem cells including activin, but does not disclose a human-type recombinant protein used in the present invention.

As described in Examples described below, only by adding a human-type recombinant protein to the medium on mesenchymal stem cells (MSC) and similar adhesion proliferation-type cells (without coating the culture substrate with the human-type recombinant protein), adhesion ability of the cells to a culture tool (6-well cell culture plate, culture bag) was improved, and further the proliferation ability of the cells was improved. Compared with a case where the culture tool was coated with a human-type recombinant protein, the adhesion and proliferation ability of the cells can be improved by a small amount of a human-type recombinant protein. As described above, the achievement of excellent cell proliferation ability by using a medium in which a human-type recombinant protein selected from laminin, collagen, gelatin, or a variant thereof is dissolved in a content of 0.01 ng/mL to 500 μg/mL was unexpectable.

According to the present invention, it was found that cell adhesion and proliferation ability can be improved by directly adding a human-type recombinant protein to a medium without using a culture tool coated with a human-type recombinant protein. According to the present invention, mass culture of cells becomes possible such that advantages in view of supplying therapeutic cells are significant and contribution to practical use of regenerative medicine is expected.

Another object of practical application of regenerative medicine is non-use of serum (serum free) and non-use of an animal-derived ingredient (Xeno free). However, since a human-type recombinant protein is used, the present invention is advantageous in this point of view.

The cell culture method as defined in the appended claims, the medium, and the medium kit of the present disclosure can be used in the manufacturing of therapeutic cells in regenerative medicine.

[1] Cell culture method

The cell culture method of the embodiment of the present invention includes culturing cells in a medium in which the added human-type recombinant protein is dissolved as defined in the appended claims.

The expression "the human-type recombinant protein is dissolved" means that the medium is liquid and 60 mass% or greater of the human-type recombinant protein, preferably 70 mass% or greater, more preferably 90 mass% or greater, and particularly preferably 100 mass% of the human-type recombinant protein is dissolved in the liquid medium.

The human-type recombinant protein used in the present invention is laminin, collagen, gelatin, or a variant thereof as defined in the appended claims.

The expression "human-type" means being derived from an amino acid sequence of a protein in humans.

A recombinant protein is a protein manufactured by gene recombination technology and specifically means a protein expressed in a host cell having a gene that encodes a protein of interest.

The variant is a protein having an amino acid sequence in which a part of the amino acid sequence of laminin, collagen, or gelatin has been modified and means a protein having the same physiological activity as the original laminin, collagen, or gelatin that is not modified. Modification means deletion, substitution, addition, and/or insertion of one to several (preferably 1 to 10, more preferably 1 to 5, and even more preferably 1 to 3) amino acid residues.

The content of the human-type recombinant protein in the medium is 0.01 ng/mL to 500 μg/mL, preferably 0.01 ng/mL to 300 μg/mL, more preferably 0.01 ng/mL to 100 μg/mL, and even more preferably 0.01 ng/mL to 10 μg/mL. The content of the human-type recombinant protein in the medium may be 0.02 ng/mL or greater, 0.03 ng/mL or greater, 0.04 ng/mL or greater, 0.05 ng/mL or greater, 0.06 ng/mL or greater, 0.07 ng/mL or greater, 0.08 ng/mL or greater, or 0.09 ng/mL or greater. The content of the human-type recombinant protein in the medium may be 1 μg/mL or less, 0.5 μg/mL or less, 0.4 μg/mL or less, 0.3 μg/mL or less, 0.2 μg/mL or less, 0.1 μg/mL or less, 0.05 μg/mL or less, 0.04 μg/mL or less, or 0.03 μg/mL or less.

In a case where the content of the human-type recombinant protein is less than 0.01 ng/mL, the cell proliferation ability decreases. The content greater than 500 μg/mL is not necessary in view of cell proliferation ability and is not advantageous in terms of cost.

According to the present invention, the human-type recombinant protein may be added to the medium, and it is not necessary to coat the culture tool with the human-type recombinant protein. In a case where the adhesion proliferation-type cells such as mesenchymal stem cells are cultured, the adhesion ability to the culture tool is improved by adding the human-type recombinant protein to the medium, and as a result, the cell proliferation ability is improved.

The content of the human-type recombinant protein in the medium can be measured by mass spectrometry such as liquid chromatograph mass spectrometry (LC-MS).

<Human-type recombinant protein>

According to the present invention as defined in the appended claims, laminin, collagen, gelatin, or a variant thereof is used as the human-type recombinant protein, but gelatin is preferable.

As the gelatin, polypeptide or a proteinous substance having an amino acid sequence similar to gelatin prepared by genetic recombination technology can be used, and preferably, recombinant gelatin having an amino acid sequence derived from the partial amino acid sequence of collagen may be used.

The recombinant gelatin preferably has a repetition of a sequence (X and Y each independently represent any amino acids) represented by Gly-X-Y which is characteristic to collagen. Here, a plurality of pieces of Gly-X-Y may be the same as or different from each other.

Examples thereof include recombinant gelatin disclosed in EP1014176, US6992172B, WO2004/85473A, and WO2008/103041A, but the recombinant gelatin is not limited thereto. Preferred recombinant gelatin used in the present disclosure is recombinant gelatin of the following aspect.

The recombinant gelatin is excellent in biocompatibility with original performance of natural gelatin, and is excellent in non-infection properties since there is no concern of bovine spongiform encephalopathy (BSE) and the recombinant gelatin with not being naturally derived. In addition, the recombinant gelatin is even compared to natural gelatin, and a sequence is determined. Therefore, it is possible to accurately design the strength and degradability so as to reduce deviation through cross-linking or the like.

The molecular weight of recombinant gelatin is not particularly limited, but is preferably 2,000 to 100,000 (2 kDa (kilodaltons) to 100 kDa), more preferably (2,500 to 95,000 (2.5 kDa to 95 kDa), still more preferably 5,000 to 90,000 (5 kDa to 90 kDa), and most preferably 10,000 to 90,000 (10 kDa to 90 kDa).

The recombinant gelatin preferably has a repetition of a sequence represented by Gly-X-Y which is characteristic to collagen. Here, a plurality of pieces of Gly-X-Y may be the same as or different from each other. In Gly-X-Y, Gly represents glycine and X and Y represent any amino acid (preferably represents any amino acid other than glycine). The sequence represented by Gly-X-Y characteristic to collagen is a partial structure which is extremely specific compared to other protein in a composition or a sequence of an amino acid of gelatin/collagen. In this section, glycine occupies about one third of the entirety of the amino acid sequence, one sequence is repeated every three sequences. Glycine is the simplest amino acid. Therefore, there is a little restraint in arrangement of molecular chains and glycine significantly contributes to regeneration of a helix structure during gelation. It is preferable that amino acids represented by X and Y contain many imino acids (proline and oxyproline) and occupy 10% to 45% of the entirety of the sequence. Preferably 80% or more of the sequence of the amino acids, more preferably 95% or more of the sequence of the amino acids, and most preferably 99% or more of the sequence of the amino acids in the recombinant gelatin have a repeating structure of Gly-X-Y.

In general gelatin, a polar amino acid with an electrical charge and a polar non-charged amino acid exist by 1:1 in polar amino acids. Here, the polar amino acid specifically indicates cysteine, aspartic acid, glutamic acid, histidine, lysine, asparagine, glutamine, serine, threonine, tyrosine, or arginine. Among these, the polar non-charged amino acid indicates cysteine, asparagine, glutamine, serine, threonine, or tyrosine. In recombinant gelatin used in the present invention, the proportion of the polar amino acid in the whole constituent amino acid is 10% to 40% and preferably 20% to 30%. The proportion of a non-charged amino acid in the polar amino acid is preferably greater than or equal to 5% and less than 20% and more preferably greater than or equal to 5% and less than 10%. Furthermore, it is preferable that any one amino acid of serine, threonine, asparagine, tyrosine, and cysteine is not included, and it is more preferable that two or more amino acids thereof are not included on a sequence. It is preferable that the recombinant gelatin used in the present invention does not include serine and threonine. The recombinant gelatin used in the present invention does not include serine, threonine, asparagine, tyrosine, and cysteine.

In general, in polypeptides, minimum amino acid sequences which work as cell adhesion signals are known (for example, Nagai Shoten Co., Ltd., "Pathophysiology", Vol. 9, No. 7 (1990) p. 527). The recombinant gelatin used in the present invention has two or more sequences of these cell adhesion signals in one molecule as defined in the appended claims. As the specific sequences, sequences such as an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence, which are represented by one-letter notation of amino acids are preferable in that there are many kinds of cells adhered. An RGD sequence, a YIGSR sequence, a PDSGR sequence, an LGTIPG sequence, an IKVAV sequence, and a HAV sequence are more preferable and an RGD sequence is particularly preferable. In the RGD sequence, an ERGD sequence is preferable.

As arrangement of RGD sequences in recombinant gelatin used in the present invention, it is preferable that the number of amino acids between RGDs is between 0 to 100 and preferably between 25 to 60 without being even.

The content of this minimum amino acid sequence is preferably 3 to 50, more preferably 4 to 30, and particularly preferably 5 to 20 in one molecule of protein. The most preferable content thereof is 12.

In recombinant gelatin used in the present invention, the proportion of RGD (Arg-Gly-Asp) motifs with respect to the total number of amino acids is preferably at least 0.4%. In a case where recombinant gelatin contains 350 or more amino acids, each stretch of the 350 amino acids preferably contains at least one RGD motif. The proportion of RGD motifs is more preferably at least 0.6%, even more preferably at least 0.8%, still even more preferably at least 1.0%, particularly preferably at least 1.2%, and most preferably at least 1.5% with respect to the total number of amino acids. The number of RGD motifs within a recombinant peptides is preferably at least 4, more preferably 6, even more preferably 8, and particularly preferably 12 to 16 per 250 amino acids. The proportion of RGD motifs being 0.4% corresponds to at least one RGD sequence per 250 amino acids. The number of RGD motifs is an integer, and therefore, gelatin formed of 251 amino acids needs to contain at least two RGD sequences in order to satisfy the characteristics of 0.4%. It is preferable that the recombinant gelatin of the embodiment of the present invention contains at least two RGD sequences per 250 amino acids, more preferably contains at least three RGD sequences per 250 amino acids, and still more preferably contains at least four RGD sequences per 250 amino acids. As a further mode of the recombinant gelatin of the embodiment of the present invention, the recombinant gelatin contains at least 4 RGD motifs, preferably 6 RGD motifs, more preferably 8 RGD motifs, and still more preferably 12 to 16 RGD motifs.

In addition, the recombinant gelatin may be partially hydrolyzed.

The recombinant gelatin used in the present invention is represented by the following formula.

A-[(Gly-X-Y)$_n$]$_m$-B

In the formula, A represents any amino acid or an amino acid sequence, B represents any amino acid or an amino acid sequence, n pieces of X each independently represent any amino acid, and n pieces of Y each independently represent any amino acid. n preferably represents an integer of 3 to 100, more preferably an integer of 15 to 70, and even more preferably an integer of 50 to 65. m preferably represents an integer of 2 to 10 and more preferably represents an integer of 3 to 5. n pieces of Gly-X-Y may be the same as or different from each other.

The recombinant gelatin used in the present invention is preferably represented by the following formula.

Gly-Ala-Pro-[(Gly-X-Y)$_{63}$]$_3$-Gly

In the formula, 63 pieces of X each independently represent any amino acid and 63 pieces of Y each independently represent any amino acid. 63 pieces of Gly-X-Y may be the same as or different from each other.

It is preferable that a plurality of sequence units of collagen which naturally exists are bonded to a repeating unit. Any naturally existing collagen referred to herein may be used as long as the collagen naturally exists, but is preferably I type collagen, II type collagen, III type collagen, IV type collagen, or V type collagen, and more preferably I type collagen, II type collagen, or III type collagen. According to another form, the above-described collagen is preferably derived from a human-type, cattle, a pig, a mouse, or a rat, and is more preferably derived from a human-type.

An isoelectric point of the recombinant gelatin used in the present invention is preferably 5 to 10, more preferably 6 to 10, and still more preferably 7 to 9.5. The measurement of the isoelectric point of the recombinant gelatin can be carried out by measuring the pH after passing a 1 mass% gelatin solution through a mixed crystal column of a cation-anion exchange resin above-described disclosed in isoelectric focusing method (refer to Maxey, C. R. (1976; Phitogr. Gelatin 2, Editor Cox, P. J. Academic, London, Engl.)).

It is preferable that the recombinant gelatin is not deaminated.

It is preferable that the recombinant gelatin does not have a telopeptide.

It is preferable that the recombinant gelatin is a substantially pure polypeptide which is prepared using a nucleic acid encoding an amino acid sequence.

The human-type recombinant protein that is recombinant gelatin particularly preferably has

(1) an amino acid sequence described in SEQ ID No: 1; or
(2) an amino acid sequence having 80% or more (preferably 90% or more, more preferably 95% or more, and particularly preferably 98% or more) sequence identity to the amino acid sequence described in SEQ ID No: 1 and has cell adhesiveness.

The human-type recombinant protein that is recombinant gelatin most preferably has the amino acid sequence described in SEQ ID No: 1.

The sequence identity of the embodiment of the present invention refers to a value calculated in the following equation.

$$\% \text{ Sequence identity} = [(\text{the number of identical residues}) / (\text{alignment length})] \times 100$$

The sequence identity between two amino acid sequences can be determined by any method well-known to those skilled in the art and can be determined by the Basic Local Alignment Search Tool (BLAST) program (J. Mol. Biol. 215: 403 to 410, 1990) or the like.

The recombinant gelatin is formed of an amino acid sequence in which one or several amino acids are deleted, substituted, or added in the amino acid sequence described in SEQ ID No: 1 and has cell adhesiveness.

"One or several" in the expression "amino acid sequence in which one or several amino acids are deleted, substituted, or added" preferably means 1 to 20 amino acids, more preferably means 1 to 10 amino acids, still more preferably means 1 to 5 amino acids, and particularly preferably means 1 to 3 amino acids.

The recombinant gelatin can be manufactured through gene recombination technology which is known to those skilled in the art, and can be manufactured in accordance with, for example, methods disclosed in EP1014176A2, US6992172B, WO2004/85473A, and WO2008/103041A. Specifically, a gene encoding an amino acid sequence of predetermined recombinant gelatin is acquired, the acquired gene is incorporated into an expression vector to manufacture a recombinant expression vector, and a transformant is manufactured by introducing the recombinant expression vector into an appropriate host. The recombinant gelatin is manufactured by culturing the obtained transformant in an appropriate medium. Therefore, it is possible to prepare the recombinant gelatin by collecting the recombinant gelatin manufactured from a culture product.

The hydrophilicity value, the "I / IOB" value, of the human-type recombinant protein used in the present invention is preferably 0 to 1.0, more preferably 0 to 0.6, and even more preferably 0 to 0.4. IOB is an index of hydrophilic and hydrophobic properties based on an organic conceptual diagram representing polarity and non-polarity of an organic compound proposed by Atsushi HUJITA, and the details thereof are described in, for example, "Pharmaceutical Bulletin", vol. 2, 2, pp. 163-173 (1954), "Area of Chemistry" vol. 11, 10, pp. 719-725 (1957), and "Fragrance Journal, vol. 50, pp. 79-82 (1981). Briefly, the root of every organic compound is set to methane ($CH_4$), and all of other compounds are regarded as derivatives of methane. Certain numerical values for the number of carbons thereof, a substituent group, a transformation portion, a ring, and the like are set, and an organic value (OV) and an inorganic value (IV) are obtained by adding the score thereof. These values are plotted on a diagram in which the organic value is represented on the X-axis and the inorganic value is represented on the Y-axis. IOB in the organic conceptual diagram refers to a ratio of the inorganic value (IV) to the organic value (OV) in the organic conceptual diagram, that is, "inorganic value (IV)/organic value (OV)". The details of the organic conceptual diagram can be referred to "New Edition Organic Conceptual Diagram -Foundation and Application-" (written by Yoshio KOUDA, Sankyo Shuppan Co., Ltd., 2008). In the present specification, the hydrophilic and hydrophobic properties are represented by a "1/IOB" value which was obtained by taking a reciprocal number of IOB. This is a notation of representing more hydrophilic properties as the "1/IOB" value becomes small (close to 0).

The hydrophilic and hydrophobic indexes of the human-type recombinant protein used in the present invention, which is represented by a grand average of hydropathicity (GRAVY) value is preferably -9.0 to 0.3, and more preferably -7.0 to 0.0. The grand average of hydropathicity (GRAVY) value can be obtained by methods of "Gasteiger E., Hoogland C., Gattiker A., Duvaud S., Wilkins M.R., Appel R.D., Bairoch A.; Protein Identification and Analysis Tools on the ExPASy Server; (In) John M. Walker (ed): The Proteomics Protocols Handbook, Humana Press (2005). pp. 571-607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31:3784-3788 (2003)".

<Cell and cell culture method>

Types of the cell cultured by the method of the embodiment of the present invention are not particularly limited as long as the cell can be cultured in the medium. The cell may be either an adherent cell or a floating cell, but an adherent cell is preferable. The adherent cell refers to a cell having a property of adhering to a culture vessel or a substrate.

The cells are preferably animal cells, more preferably vertebrate-derived cells, and particularly preferably human-type-derived cells. The types of vertebrate-derived cells (particularly, human-type-derived cells) may be any of universal cells, somatic stem cells, precursor cells, and mature cells.

It is possible to use, for example, embryonic stem (ES) cells, germ-stem (GS) cells, or artificial pluripotent stem (iPS) cells as the universal cells.

It is possible to use, for example, mesenchymal stem cells (MSC), hematopoietic stem cells, amniotic cells, umbilical cord blood cells, bone marrow-derived cells, myocardial stem cells, adipose-derived stem cells, or neural stem cells can be used as the somatic stem cell.

It is possible to use, for example, skin, dermis, epidermis, muscle, cardiac muscles, nerves, bones, cartilage, endothelium, brain, epithelium, heart, kidney, liver, pancreas, spleen, oral cavity, cornea, bone marrow, umbilical cord blood, amnion, or cells derived from hair as the precursor cells and the mature cells.

It is possible to use, for example, ES cells, iPS cells, MSCs, chondrocytes, osteoblasts, osteoprecursor cells, mesenchymal cells, myoblasts, cardiac muscle cells, cardiomyoblasts, nerve cells, hepatocytes, beta cells, fibroblasts, corneal endothelial cells, vascular endothelial cells, corneal epithelial cells, amniotic cells, umbilical cord blood cells, bone marrow-derived cells, or hematopoietic stem cells as the human-type-derived cells.

In a case where the cultured cells are used in transplantation, the cells may be derived from any of autologous cells and heterologous cells.

Culturing of cells can be carried out optionally in a $CO_2$ incubator $CO_2$ (5% $CO_2$ incubator), and the culture can be carried out generally at 30°C to 45°C, preferably at 35°C to 40°C (for example, 37°C) for 1 hour to 72 hours, preferably for 1 hour to 24 hours, more preferably for 1 hour to 12 hours, and still more preferably for 2 hours to 8 hours. Depending on the situation of cell proliferation, cells can be cultured for more than 72 hours. The culture may be stationary culture or shake culture.

[2] Medium and medium kit

The present disclosure provides a medium including a basal medium component and a dissolved human-type recombinant protein, in which the human-type recombinant protein is laminin, collagen, gelatin, or a variant thereof, and the content of the human-type recombinant protein in the medium is 0.01 ng/mL to 500 $\mu$g/mL.

The present disclosure further provides a medium kit including a basal medium component and a human-type recombinant protein in a separated manner, in which the human-type recombinant protein is laminin, collagen, gelatin, or a variant thereof, and the content of the human-type recombinant protein in the medium manufactured by mixing the basal medium component and the human-type recombinant protein is 0.01 ng/mL to 500 $\mu$g/mL.

The human-type recombinant protein and preferable embodiments thereof are as described above in the present specification.

The basal medium is not particularly limited, but examples thereof include Dulbecco's Modified Eagle's Medium (DMEM), Eagle's Minimum Essential Medium (MEM), F12, Ham, RPMI 1640, MCDB (MCDB102, 104, 107, 131, 153, 199, and the like), L15, SkBM (registered trademark), RITC80-7, MesenPro (Life Technologies Corporation). Many of these basal media are commercially available.

As the basal medium component, the above basal medium may be used in a standard composition without change (for example, in a commercially available state without change), and the composition thereof may be appropriately changed depending on cell types and cell conditions. Accordingly, the basal medium component is not limited to a component having the well-known composition, and one or more components may be added, removed, increased, or decreased.

The amino acid included in the basal medium component is not particularly limited, and examples thereof include L-arginine, L-cystine, L-glutamine, Glycine, L-histidine, L-isoleucine, L-leucine, L-lysine, L-methionine, L-phenylalanine, L-serine, L-threonine, L-tryptophan, L-tyrosine, and L-valine.

Vitamins included in the basal medium component are not particularly limited, and examples thereof include calcium D-pantothenate, choline chloride, folic acid, i-inositol, niacinamide, riboflavin, thiamine, pyridoxine, biotin, lipoic acid, vitamin $B_{12}$, adenine, and thymidine.

The electrolyte included in the basal medium component is not particularly limited, and examples thereof include $CaCl_2$, KCl, $MgSO_4$, NaCl, $NaH_2PO_4$, $NaHCO_3$, $Fe(NO_3)_3$, $FeSO_4$, $CuSO_4$, $MnSO_4$, $Na_2SiO_3$, $(NH_4)6Mo_7O_{24}$, $NaVO_3$, $NiCl_2$, and $ZnSO_4$.

In addition to these components, the basal medium component may contain saccharides such as D-glucose, a pH indicator such as sodium pyruvate and phenol red, putrescine, antibiotics, and the like.

The medium of the present disclosure may be a medium including serum or a medium not including serum. The content of the serum in the medium of the present disclosure is preferably 0 volume% to 20 volume%, more preferably 0 volume% to 10 volume%, even more preferably 0 volume% to 5 volume%, and particularly preferably 0 volume% to 2 volume%.

In a case where it is assumed that the cells are applied to humans, it is preferable that the medium does not substantially include heterologous serum components. Here, the "heterologous serum component" means a serum component derived from an organism of a species different from the recipient. For example, in a case where the recipient is a human, serum derived from cattle and horse, such as fetal bovine serum (FBS, FCS), calf serum (CS), and equine serum (HS), corresponds to a heterologous serum component.

The present invention will be more specifically described using the following examples, but is not limited by the examples.

Examples

(1) Recombinant Gelatin

The following CBE3 (which is disclosed in WO2008/103041A) was used as recombinant gelatin.
CBE3:

Molecular weight: 51.6 kD
Structure: GAP[(GXY)$_{63}$]$_3$G
Number of amino acids: 571
RGD sequence: 12

Imino acid content: 33%

Almost 100% of amino acids have a repeating structure of GXY. In the amino acid sequence of CBE3, serine, threonine, asparagine, tyrosine, and cysteine are not included. CBE3 has an ERGD sequence.

Isoelectric point: 9.34
GRAVY value: -0.682
1/IOB value: 0.323

Amino acid sequence (SEQ ID No: 1 in a sequence table) (which is the same as that of SEQ ID No: 3 in WO20081103041A. However, X in the end is corrected to "P").

GAP(GAPGLQGAPGLQGMPGERGAAGLPGPKGERGDAGPKGADGAPGAPGL

QGMPGERGAAGLPGPKGERGDAGPKGADGAPGKDGVRGLAGPIGPPGERGAAGLPG

PKGERGDAGPKGADGAPGKDGVRGLAGPIGPPGPAGAPGAPGLQGMPGERGAAGLP

GPKGERGDAGPKGADGAPGKDGVRGLAGPP)3G

(2) Measurement of coat amount of CBE3

In the following experiments, the coat amount of CBE3 was measured according to the method described in EP0138092A2. As the sample, amino acid obtained by adding NaOH to the plate coated with CBE3 and performing a treatment at 50°C for 6 hours or more was used.

[Example 1] Cell Proliferation at Passage 5 Using Cartilage-Derived Cell Yub2478 (5 Weeks)

• Level

Level 1: CBE 3 coat (coat amount: 5.7 μg/well)
Level 2: Addition of CBE3 (addition amount: 5.7 μg/well, addition of same amount as in case of coating)
Level 3: Addition of CBE3 (addition amount: 57 ng/well, addition of 1/100 amount in case of coating)
Level 4: Without addition of CBE3 (control)

• Material

Cells used: Yub2478 (cartilage-derived cells established at the National Center for Child Health and Development (hereinafter referred to as "Growing Research Laboratory") Yub2478 was established by a method in conformity with a reference document (Nasu, Takayama S, Umezawa A. Endochondral ossification model system: designed cell fate of human-type epiphyseal chondrocytes during long-term implantation. J. Cell Physiol. 2015; 230: 1376 to 1388).
Medium used: MesenPro RS (containing 2 volume% fetal bovine serum (FBS), Life Technologies Corporation)
Culture vessel: 6-well tissue culture (TC) plate (Falcon)

• Method

CBE3 coating method:
2 mL of cellnest (registered trademark) (0.1 mass% CBE3 aqueous solution, Fujifilm Corporation) was added dropwise to each well of the plate, and the plate was allowed to stand in a 37°C incubator for two hours. Cellnest (registered trademark) was removed from each well by aspiration, 6 mL of phosphate buffered saline (PBS) was added dropwise to each well, and the plate was left for 30 minutes. PBS was removed from each well by aspiration, and the plate was kept refrigerated until use. 2 mL of the medium was added dropwise to each well of the plate, cells were seeded, and cultivation was started. The cell seeding amount was $0.2 \times 10^6$ cell/well.

CBE 3 addition method:
2 mL of the medium was added dropwise to each well, and then cellnest (registered trademark) (0.1 mass% CBE3 aqueous solution, Fujifilm Corporation) was added dropwise to each well to a predetermined concentration. PBS in the same amount of cellnest (registered trademark) was added dropwise to the control well (without addition of CBE3). After the solution was homogenized in the well by pipetting, cells were seeded to start cultivation. The cell seeding amount was $0.2 \times 10^6$ cells/well.

Cell culture method:

The plates were stored in an incubator at a concentration of 37°C/5% $CO_2$ after the start of culture by the above protocol. After one week has passed, the cells were peeled off by using a trypsin solution (Wako Pure Chemical Industries, Ltd.), and the number of cells was counted by using Vi-Cell (Becton, Dickinson and Company, hereinafter also referred to as "BD"). A plate was newly prepared and the cells were seeded again ($0.2 \times 10^6$ cell/well) to be subcultured, and the plate was stored in an incubator at a concentration of 37°C/5% $CO_2$.

After one week, operations of peeling off the cells with the trypsin solution, measuring the number of cells, subculturing the cells, and storing the plate in an incubator at a concentration of 37°C/5% $CO_2$ were repeated up to 5 passages.

• Calculation method

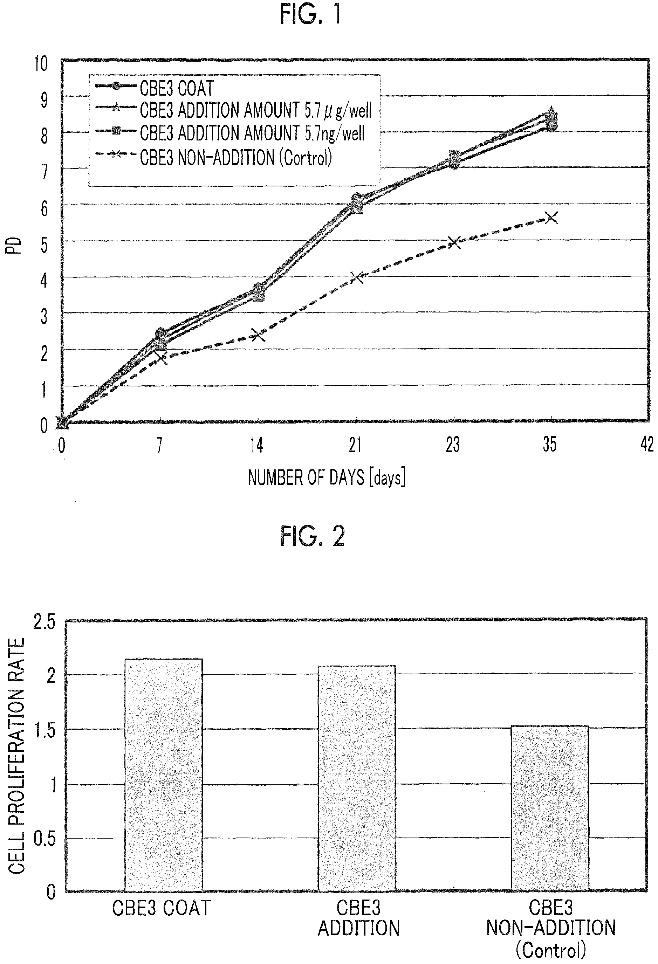

The cell proliferation curve obtained by calculating PD (the number of cell divisions) by the following formula is illustrated in Fig. 1.

$$\text{PD (the number of cell divisions)} = \text{PD value at the previous passage} + \log_2 (\text{the number of measured cells / the number of seeded cells})$$

• Result

Compared with Level 4, the proliferation was improved at all of Levels 1 to 3. There was almost no difference in proliferation in Levels 1 to 3, and the effect did not change even in a case where CBE3 addition is reduced to 1/100 as compared with CBE3 coat.

[Example 2] Cell proliferation rate during short-term culture (after one day of culture to after three days of culture) using the cartilage-derived cells Yub2478

• Level

Level 1: CBE 3 coat (coat amount: 5.7 μg/well)
Level 2: Addition of CBE3 (addition amount: 57 ng/well, addition of 1/100 amount in case of coating)
Level 3: Without addition of CBE3 (control)

• Material

Cells used: Yub2478 (cartilage-derived cells established at Growing Research Laboratory)
Medium used: MesenPro RS (containing 2 volume% fetal bovine serum (FBS), Life Technologies Corporation)
Culture vessel: 6-well tissue culture (TC) plate (Falcon)

• Method

The CBE3 coating method was performed in the same manner as in Example 1.
The CBE3 addition method was performed in the same manner as in Example 1.
The cell seeding amount was $0.2 \times 10^6$ cell/well.

Cell culture method:

The plates were stored in an incubator at a concentration of 37°C/5% $CO_2$ after the start of culture by the above protocol. Two plates were prepared for each level. After a predetermined period of time, the cells were peeled off by using a trypsin solution (Wako Pure Chemical Industries, Ltd.), and the number of cells was measured for one plate after one day and the other one plate after three days by using Vi-Cell (BD).

• Calculation method

The cell proliferation rate obtained by calculation with the following formula is illustrated in Fig. 2.

$$\text{Cell proliferation rate} = \text{the number of cells after three days of culture / the number of cells after one day of culture}$$

• Result

Compared with Level 3, the proliferation rates were improved at both of Levels 1 to 2. There was almost no difference in proliferation in Levels 1 to 2, and the effect did not change between CBE3 coat and CBE3 addition (1/100 amount).

[Example 3] Cell adhesion ability to surface of a culture bag using bone marrow-derived cells

• Level

Level 1: Addition of CBE3 (addition amount 1: 57 ng/well)
Level 2: Without addition of CBE3 (control)

• Material

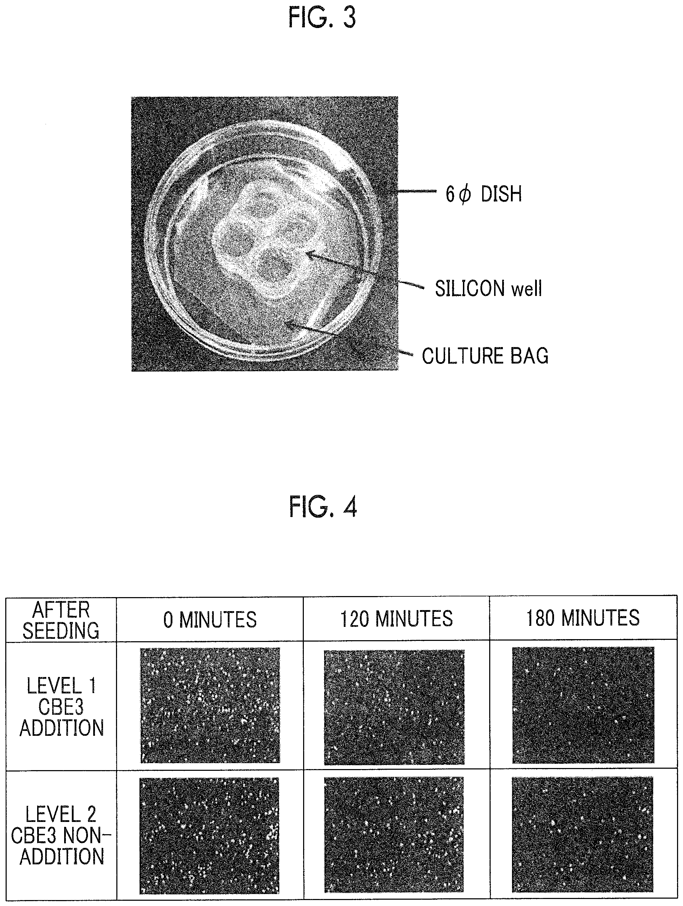

Cells used: Bone marrow-derived stem cells (Bone marrow-derived stem cells: BMSC) (Bone marrow-derived cell, Lonza) (trade name HMSC, catalog number PT-2501)
Medium used: MesenPro RS (containing 2 volume% fetal bovine serum (FBS), Life Technologies Corporation)
Culture vessel: Place a sheet obtained by cutting off a culture bag (Nipro Corporation) on a dish (Falcon) of 6 φ (diameter 6 cm) such that the inside of the bag is on top, and then a silicon well (SARSTEDT AG & Co. KG) with a hole (no bottom) was brought into close contact thereto to obtain a culture vessel (Fig. 3).
Observation device: fluorescence microscope BZ-X710 (Keyence Corporation)

• Method

CBE 3 addition method:
0.5 mL of a medium was added dropwise to each well. Cellnest (registered trademark) (0.1 mass% CBE3 aqueous solution, Fujifilm Corporation) was added dropwise to each well to a predetermined concentration. A PBS buffer in the same amount of cellnest (registered trademark) was added dropwise to the control well (without addition of CBE3). After the solution was homogenized in the well by pipetting, cells were seeded to start cultivation. The cell seeding amount was $0.05 \times 10^6$ cell/well.
Cell culture method:
After cultivation was started under the above protocol, the culture vessel was placed in a fluorescence microscope chamber in an environment at a concentration of 37°C/5% $CO_2$, and the chamber lid was closed. The visual field to be observed was positioned so that the number of cells in the visual field was almost the same. The well was imaged by one sheet per 5 minutes, and the imaging was repeated until 180 minutes. The number of cells that were not adhered was counted from the images at each time (the adhered cells appeared black and were not counted). Images of cells at 0 minutes, 120 minutes, and 180 minutes are illustrated in Fig. 4. White spots indicate cells that are not adhered, and spots that are entirely black and of which the circumferences remain slightly white are adhering cells.

• Calculation method

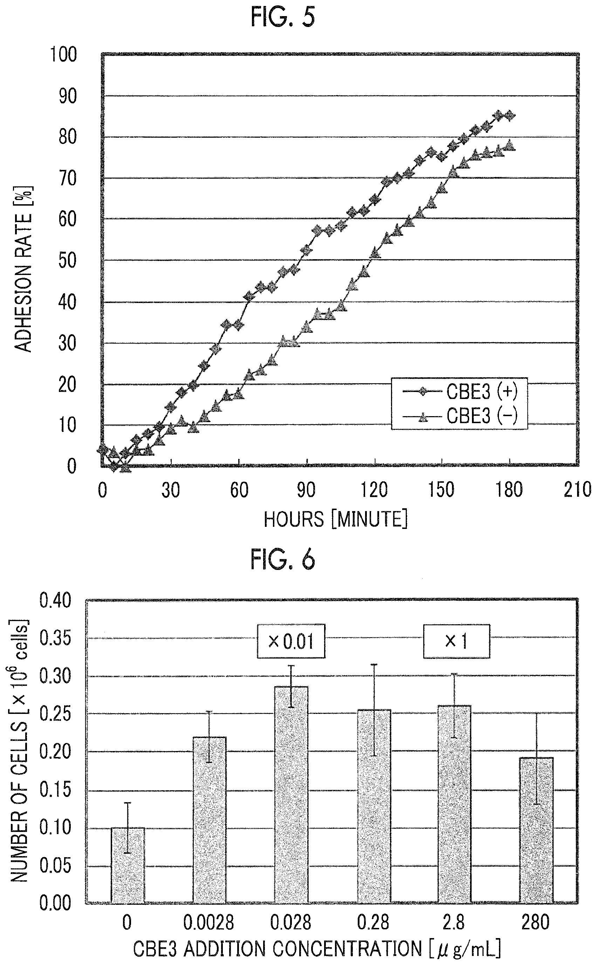

The adhesion rate obtained by calculation with the following formula is illustrated in Fig. 5.

$$\text{Adhesion rate } [\%] = (1 - \text{measurement value of the number of cells at that time} / \text{maximum measurement value of the number of cells}) \times 100$$

• Result

Adherence at Level 1 is satisfactory compared with Level 2, and cells are quickly adhered to the surface of the culture bag by adding CBE3.

[Example 4] Concentration dependence in a case of adding CBE3

Cell proliferation during short-term culture (after seven days of culture) using the cartilage-derived cells Yub2505

• Level

Level 1: Without addition of CBE3
Level 2: CBE3 addition amount (0.0028 $\mu$g/mL)
Level 3: CBE3 addition amount (0.028 $\mu$g/mL)
Level 4: CBE3 addition amount (0.28 $\mu$g/mL)
Level 5: CBE3 addition amount (2.8 $\mu$g/mL)
Level 6: CBE3 addition amount (280 $\mu$g/mL)

• Material

Cells used: Yub2505 (cartilage-derived cells established at Growing Research Laboratory) Yub2505 was established by a method in conformity with a reference document (Nasu, Takayama S, Umezawa A. Endochondral ossification model system: designed cell fate of human-type epiphyseal chondrocytes during long-term implantation. J. Cell Physiol. 2015; 230: 1376 to 1388).
Medium used: MesenPro RS (containing 2 volume% fetal bovine serum (FBS), Life Technologies Corporation)
Culture vessel: 6-well tissue culture (TC) plate (Falcon)

• Method

The CBE3 addition method was performed in the same manner as in Example 1. The cell seeding amount was $0.1 \times 10^6$ cell/well.
Cell culture method:
The plates were stored in an incubator at a concentration of 37°C/5% $CO_2$ after the start of culture by the above protocol. After seven days, the cells were peeled off by using a trypsin solution (Wako Pure Chemical Industries, Ltd.), and the number of cells was counted by using Vi-Cell (BD).

• Result

Calculation results of the number of cells are presented in Fig. 6.
Compared with Level 1, the proliferation was satisfactory at Levels 2 to 6. At Level 3 in which the addition amount of CBE3 was 0.028 $\mu$g/mL and the subsequent levels, there was almost no difference in proliferation ability improvement, and at Level 6 in which the addition amount of CBE3 was 280 μg/mL, compared with Levels 3 to 5, the proliferation ability improvement effects were rather decreased. Here, compared with Level 1 in which CBE3 was not added, Level 6 maintained satisfactory proliferation.

[Example 5] Concentration dependence in a case of adding CBE3

Cell proliferation during short-term culture (after seven days of culture) using the bone marrow-derived cells BMSC

• Level

Level 1: Without addition of CBE3
Level 2: CBE3 addition amount (0.00009 μg/mL=0.09 ng/mL)
Level 3: CBE3 addition amount (0.028 μg/mL)
Level 4: CBE3 addition amount (513 μg/mL)
Level 5: CBE3 addition amount (627 μg/mL)

• Material

Cell used: Bone marrow-derived stem cell (BMSC) (bone marrow-derived cell, Lonza) (trade name HMSC, catalog number PT-2501)
Medium used: MesenPro RS (containing 2 volume% fetal bovine serum (FBS), Life Technologies Corporation)
Culture vessel: Dish of 6φ (diameter 6 cm) (Sumitomo Bakelite Company Limited)

• Method

The CBE3 addition method was performed in the same manner as in Example 1. The cell seeding amount was $0.15 \times 10^6$ cell/well.
• Cell culture method:
The dishes were stored in an incubator at a concentration of 37°C/5% $CO_2$ after the start of culture by the above protocol. After seven days, the cells were peeled off by using a trypsin solution (Wako Pure Chemical Industries, Ltd.), and the number of cells was counted by using Vi-Cell (BD).

• Result

Calculation results of the number of cells are presented in Table 1.
In the determination, A: the number of cells $0.33 \times 10^6$ cells or greater, B: the number of cells $0.30 \times 10^6$ cells or greater and less than $0.33 \times 10^6$ cells, C: $0.27 \times 10^6$ cells or greater and less than $0.30 \times 10^6$ cells, and D: less than $0.27 \times 10^6$ cells.
Compared with Level 1, the proliferation was satisfactory at Levels 2 to 3. At Levels 4 and Level 5, the proliferation ability improvement effect was decreased.

[Table 1]

| level | CBE3 Concentration [μg/mL] | The number of cells [106 cell] | Determination |
|---|---|---|---|
| 1 | 0 | 0.27 | C |
| 2 | 0.00009 | 0.34 | A |
| 3 | 0.028 | 0.33 | A |
| 4 | 513 | 0.29 | C |

(continued)

| level | CBE3 Concentration [μg/mL] | The number of cells [106 cell] | Determination |
|---|---|---|---|
| 5 | 627 | 0.23 | D |

[Reference Example 1]: Comparison of Cell Proliferation of CBE3 Coat and Fibronectin Coat

Cell proliferation (5 weeks) at 5 passages was compared by using bone marrow-derived cells UDE BM.

• Level

Level 1: CBE 3 coat (coat amount: 5.7 μg/well)
Level 2: Fibronectin Coat (Commercially available product: BIOCOAT, Falcon)

• Material

Cells used: UDE BM (bone marrow-derived cells established at Growing Research Laboratory) UDE BM was established in the following method.
Bone marrow fluid was removed from the bone, and cells were collected by density gradient centrifugation. The collected cells were washed with PBS, seeded in DMEM (containing 10% FBS), and cultured. Cells adhering to the culture vessel were collected.
Medium used: MesenPro RS (containing 2 volume% fetal bovine serum (FBS), Life Technologies Corporation)
Culture vessel:

Level 1: 6-well tissue culture (TC) plate (Falcon)
Level 2: Commercially available product: BIOCOAT (Fibronectin coated product was used without change, Falcon)

• Method

CBE3 coating method:
2 mL of cellnest (registered trademark) (0.1 mass% CBE3 aqueous solution, Fujifilm Corporation) was added dropwise to each well of the plate, and the plate was allowed to stand in a 37°C incubator for two hours. Cellnest (registered trademark) was removed from each well by aspiration, 6 mL of PBS was added dropwise to each well, and the plate was left for 30 minutes. PBS was removed from each well by aspiration, and the plate was kept refrigerated until use. 2 mL of the medium was added dropwise to each well of the plate, cells were seeded, and cultivation was started. The cell seeding amount was $0.2 \times 10^6$ cell/well.
Cell culture method:
The plates were stored in an incubator at a concentration of 37°C/5% $CO_2$ after the start of culture by the above protocol. After one week has passed, the cells were peeled off by using a trypsin solution (Wako Pure Chemical Industries, Ltd.), and the number of cells was counted by using Vi-Cell (BD). A plate was newly prepared and the cells were seeded again ($0.2 \times 10^6$ cell/well) to be subcultured, and the plate was stored in an incubator at a concentration of 37°C/5% $CO_2$. After one week, operations of peeling off the cells with the trypsin solution, measuring the number of cells, subculturing the cells, and storing the plate in an incubator at a concentration of 37°C/5% $CO_2$ were repeated up to 5 passages.

• Calculation method

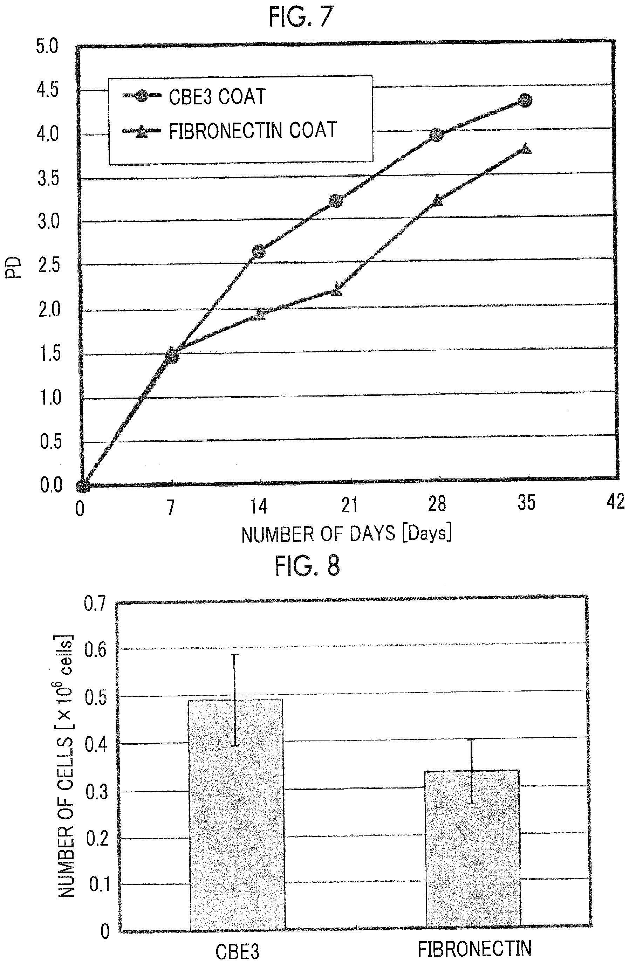

The cell proliferation curve obtained by calculating PD (the number of cell divisions) by the following formula is illustrated in Fig. 7.

$$PD \text{ (the number of cell divisions)} = PD \text{ value at the previous passage} + \log_2 \text{ (the number of measured cells / the number of seeded cells)}$$

• Result

The proliferation at Level 1 was satisfactory compared with Level 2, and CBE3 exhibits more satisfactory proliferation compared with Fibronectin commonly used as a coating material.

[Reference Example 2] Comparison of Cell Proliferation of CBE3 Coat and Fibronectin Coat

Cell proliferation during short-term culture (after one day of culture) was compared by using the cartilage-derived cells Yub2478.

• Level

Level 1: CBE 3 coat (coat amount: 5.7 $\mu$g/well)
Level 2: Fibronectin Coat (Commercially available product: BIOCOAT, Falcon)

• Material

Cells used: Yub2478 (cartilage-derived cells established at Growing Research Laboratory),
Medium used: DMEM/F12 medium (addition of 10 volume% fetal bovine serum (FBS))

Culture vessel:

Level 1: 6-well tissue culture (TC) plate (Falcon)
Level 2: Commercially available product: BIOCOAT (Fibronectin coated product was used without change, Falcon)

• Method

CBE3 coating method:
2 mL of cellnest (registered trademark) (0.1 mass% CBE3 aqueous solution, Fujifilm Corporation) was added dropwise to each well of the plate, and the plate was allowed to stand in a 37°C incubator for two hours. Cellnest (registered trademark) was removed from each well by aspiration, 6 mL of PBS was added dropwise to each well, and the plate was left for 30 minutes. PBS was removed from each well by aspiration, and the plate was kept refrigerated until use. 2 mL of the medium was added dropwise to each well of the plate, cells were seeded, and cultivation was started. The cell seeding amount was 0.2 $\times$ 10$^6$ cell/well.
Cell culture method:
The plates were stored in an incubator at a concentration of 37°C/5% $CO_2$ after the start of culture by the above protocol. After one day, the cells were peeled off by using a trypsin solution (Wako Pure Chemical Industries, Ltd.), and the number of cells was counted by using Vi-Cell (BD).

• Result

Calculation results of the number of cells are presented in Fig. 8.
Compared with Level 2, the proliferation was improved at Level 1.

[Reference Example 3] Gene expression difference between the addition or non-addition of CBE3

Cell proliferation and gene expression during short-term culture (after eight days of culture) using the bone marrow-derived cells BMSC

• Level

Level 1: Without addition of CBE3
Level 2: CBE3 addition amount (0.028 $\mu$g/mL)

• Material

Cell used: Bone marrow-derived stem cell (BMSC) (bone marrow-derived cell, Lonza) (trade name HMSC, catalog number PT-2501)
Medium used: MesenPro RS (containing 2 volume% fetal bovine serum (FBS), Life Technologies Corporation)
Culture vessel: Dish of 10 $\varphi$ (diameter 10cm) (Sumitomo Bakelite Company Limited)

• Method

The CBE3 addition method was performed in the same manner as in Example 1. The cell seeding amount was $0.4 \times 10^6$ cell/well.

• Cell culture method:

The dishes were stored in an incubator at a concentration of 37°C/5% $CO_2$ after the start of culture by the above protocol. After eight days, the cells were peeled off by using a trypsin solution (Wako Pure Chemical Industries, Ltd.), and the number of cells was counted by using Vi-Cell (BD).

• mRNA extraction method

After the number of the cells were counted with the above protocol, the supernatant was removed by centrifugation (1,000 rpm, 5 minutes) using the remaining cells used for the count. Phosphate buffered saline (PBS) was added and suspended, transferred to an Eppendorf tube (Eppendorf Corporate), centrifuged again (2,000 rpm, 5 minutes) to remove the supernatant, and stored at -80°C as a cell pellet. MRNA extraction from cell pellets was performed by using RNeasy Plus MiniKit (Qiagen, 74134) and QIA Shredder (Qiagen, 79656). The extracted mRNA was assayed by measuring the absorbance at 260 nm and 280 nm by using Nano Drop ND-1000 (Thermo Fisher Scientific).

• cDNA preparation method

The extracted mRNA, primer, and dNTP (Thermo Fisher Scientific, 18427-013) were mixed and annealed by using a ProFlex™ PCR system (Life Technologies), and PCR reaction was performed by using 5 $\times$ FS buffer, 0.1 M DTT, RNase OUT, Super Script IV Reverse Transcriptase (all are Invitrogen) so as to obtain cDNA. cDNA prepared in the same manner as the above protocol was tested.

• Gene expression profile

Differences in gene expression profiles were checked by using cDNA prepared from the cells cultured at Levels 1 and 2. Gene expression by RTPCR was checked by using Human-type Extracellular Matrix & Cell Adhesion Molecules (PAHS-013Z) and Human-type Cell Cycle (PAHS-020Z) of RT$^2$ Profiler™ PCR Arrays (Qiagen).

• Result

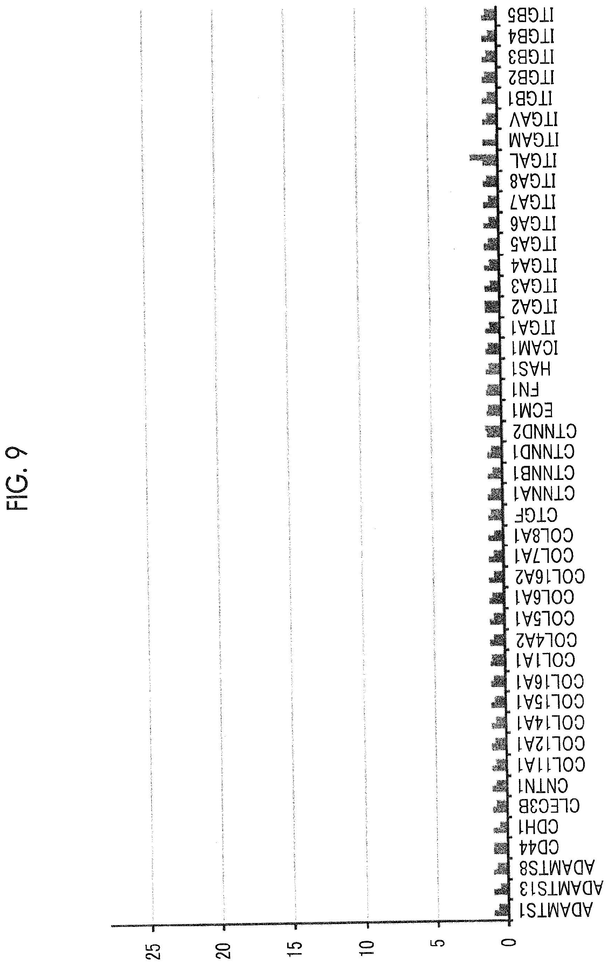

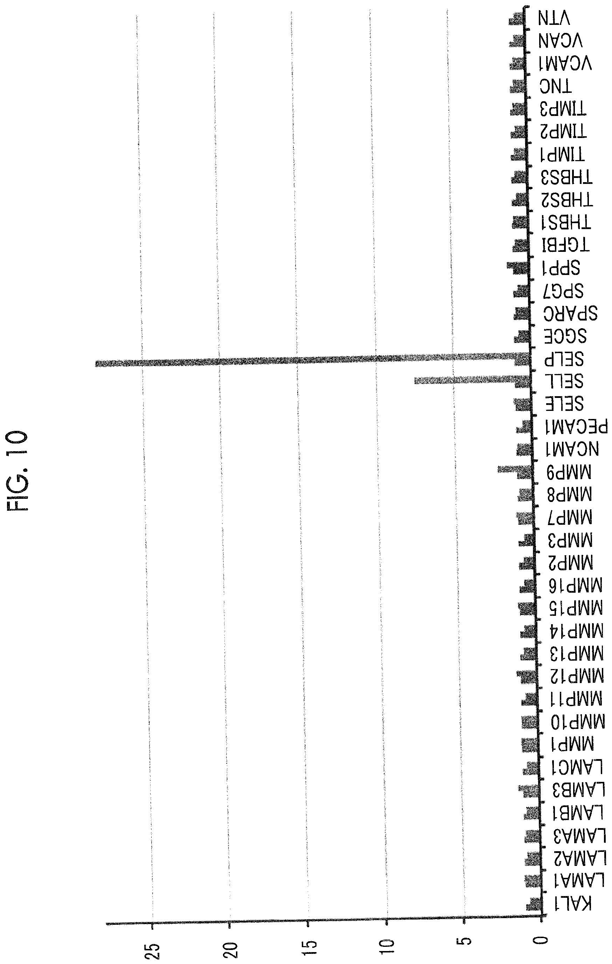

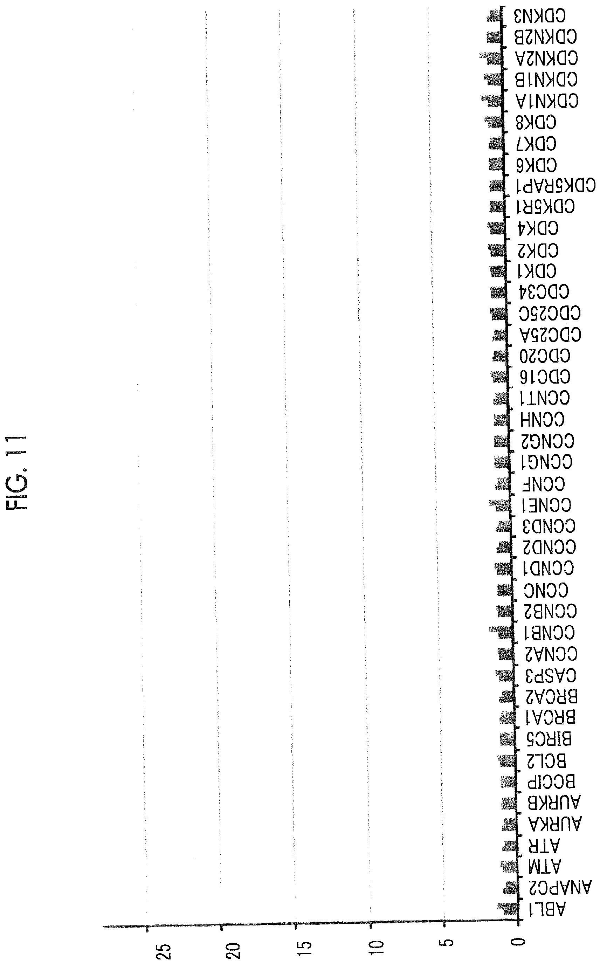

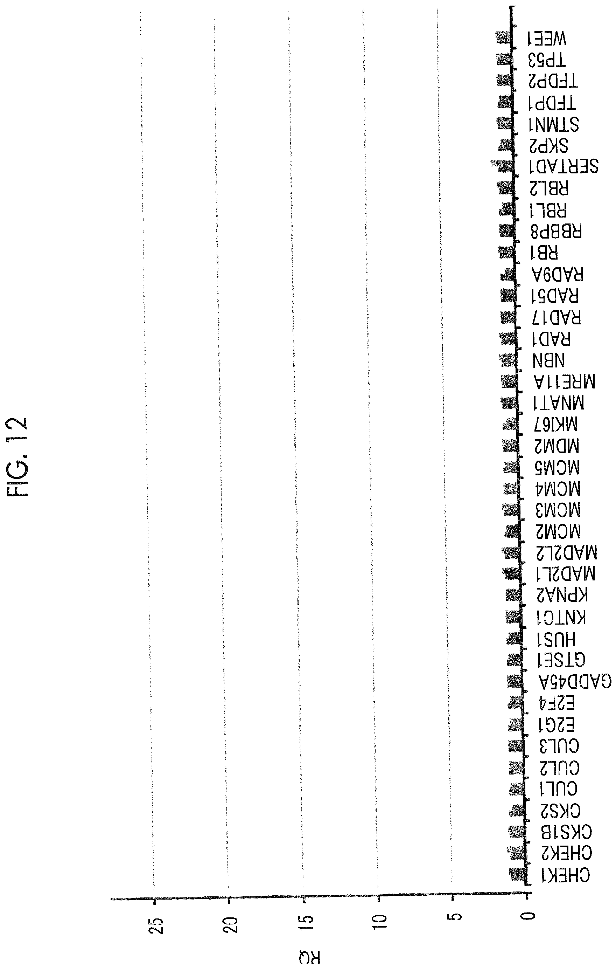

The gene expression profiles of the extracellular matrix and the cell adhesion-related gene group are illustrated in Figs. 9 and 10, and the gene expression profiles of the cell cycle-related gene group are illustrated in Figs. 11 and 12. In Figs. 9 to 12, a bar graph on the left side of each gene column represents Level 1 (without addition of CBE3), and a bar graph on the right side of each gene column represents Level 2 (addition of CBE3). In the extracellular matrix and cell adhesion related genes, genes in which expression is increased by the addition of CBE3 were found, but an expression change in the cell cycle-related gene group was rarely found.

[SEQUENCE LISTING] A cell culture method, medium and medium kit of an international application 17F00470 under the Patent Cooperation Treaty (PCT) JP17018657 20170518-00130226551701063896201705181433202017050109114386690_P1AP101_17 _1.app

## Claims

1. A cell culture method comprising:

   culturing cells at 30 to 45°C in a medium in which a human-type recombinant protein is dissolved,
   wherein the human-type recombinant protein is laminin, collagen, gelatin, or a variant thereof, and the content of the human-type recombinant protein in the medium is 0.01 ng/mL to 100 µg/mL,
   wherein the human-type recombinant protein includes recombinant gelatin having an amino acid sequence derived from a partial amino acid sequence of collagen,
   wherein the human-type recombinant protein has a repeating sequence represented by Gly-X-Y characteristic to collagen, X and Y each independently represent any one of amino acid, a plurality of pieces of the Gly-X-Y may be the same as or different from each other, and the human-type recombinant protein includes two or more sequences of cell adhesion signals in one molecule,
   wherein the cell adhesion signal is an amino acid sequence represented by Arg-Gly-Asp,
   and wherein the amino acid sequence of the human-type recombinant protein is represented by the following formula,

   A-[(Gly-X-Y)n]m-B

   in the formula, A represents any amino acid or an amino acid sequence, B represents any amino acid or an amino acid sequence, n pieces of X each independently represent any amino acid, n pieces of Y each independently represent any amino acid, and n represents an integer of 3 to 100, m represents an integer of 2 to 10, and n pieces of Gly-X-Y may be the same as or different from each other,
   wherein the cells are mesenchymal stem cells,
   and
   wherein the human-type recombinant protein has (1) an amino acid sequence described in SEQ ID No: 1, or (2) an amino acid sequence having 80% or more sequence identity to the amino acid sequence described in SEQ ID No: 1 and has cell adhesiveness.

**2.** The cell culture method according to of claim 1, wherein the molecular weight of the human-type recombinant protein is 2 kDa to 100 kDa.

**3.** The cell culture method according to any one of claims 1 or 2, wherein the molecular weight of the human-type recombinant protein is 10 kDa to 90 kDa.

**4.** The cell culture method according to any one of claims 1 to 3, wherein the amino acid sequence of the human-type recombinant protein is represented by the following formula,

$$\text{Gly-Ala-Pro-}[(\text{Gly-X-Y})_{63}]_3\text{-Gly}$$

in the formula, 63 pieces of X each independently represent any amino acid, 63 pieces of Y each independently represent any amino acid, and 63 pieces of Gly-X-Y may be the same as or different from each other.

**Patentansprüche**

**1.** Verfahren zum Kultivieren von Zellen, umfassend:

das Kultivieren von Zellen bei 30 bis 45°C in einem Medium, in dem ein rekombinantes Protein vom menschlichen Typ gelöst ist,
wobei das rekombinante Protein vom menschlichen Typ Laminin, Kollagen, Gelatine oder eine Variante davon ist, und
der Gehalt des rekombinanten Proteins vom menschlichen Typ in dem Medium 0,01 ng/mL bis 100 $\mu$g/mL beträgt,
wobei das rekombinante Protein vom menschlichen Typ rekombinante Gelatine enthält, die eine Aminosäuresequenz hat, die von einer Teilaminosäuresequenz von Kollagen abgeleitet ist,
wobei das rekombinante Protein vom menschlichen Typ eine sich wiederholende Sequenz hat, repräsentiert durch Gly-X-Y, charakteristisch für Kollagen, X und Y beide unabhängig voneinander irgendeine Aminosäure repräsentieren, eine Vielzahl der Teile der Gly-X-Y einander gleich oder verschieden voneinander sein können, und das rekombinante Protein vom menschlichen Typ zwei oder mehr Sequenzen von Zelladhäsionssignalen in einem Molekül enthält,
wobei das Zelladhäsionssignal eine Aminosäuresequenz ist, die durch Arg-Gly-Asp repräsentiert ist,
und wobei die Aminosäuresequenz des rekombinanten Proteins vom menschlichen Typ durch die folgende Formel repräsentiert ist,

$$\text{A-}[(\text{Gly-X-Y})\text{n}]\text{m-B}$$

in dieser Formel repräsentiert A irgendeine Aminosäure oder eine Aminosäuresequenz, repräsentiert B irgendeine Aminosäure oder eine Aminosäuresequenz, n Teile von X repräsentieren jeweils unabhängig voneinander irgendeine Aminosäure, n Teile von Y repräsentieren jeweils unabhängig voneinander irgendeine Aminosäure, und n repräsentiert eine ganze Zahl von 3 bis 100, m repräsentiert eine ganze Zahl von 2 bis 10, und n Teile von Gly-X-Y können einander gleich oder verschieden voneinander sein,
wobei die Zellen mesenchymale Stammzellen sind,
und
wobei das rekombinante Protein vom menschlichen Typ (1) eine Aminosäuresequenz beschrieben in SEQ ID NO: 1 hat, oder (2) eine Aminosäuresequenz mit 80% oder höherer Sequenzidentität zu der Aminosäuresequenz beschrieben in SEQ ID NO: 1 und Zelladhäsion hat.

**2.** Verfahren zum Kultivieren von Zellen gemäß Anspruch 1,
wobei das Molekulargewicht des rekombinanten Proteins vom menschlichen Typ 2 kDa bis 100 kDa beträgt.

**3.** Verfahren zum Kultivieren von Zellen gemäß irgendeinem der Ansprüche 1 oder 2,
wobei das Molekulargewicht des rekombinanten Proteins vom menschlichen Typ 10 kDa bis 90 kDa beträgt.

**4.** Verfahren zum Kultivieren von Zellen gemäß irgendeinem der Ansprüche 1 bis 3,

wobei die Aminosäuresequenz des rekombinanten Proteins vom menschlichen Typ durch die folgende Formel repräsentiert ist,

Gly-Ala-Pro-[(Gly-X-Y)$_{63}$]$_3$-Gly

in der Formel repräsentieren 63 Teile von X jeweils unabhängig voneinander irgendeine Aminosäure, repräsentieren 63 Teile von Y jeweils unabhängig voneinander irgendeine Aminosäure, und 63 Teile von Gly-X-Y können einander gleich oder verschieden voneinander sein.

**Revendications**

1. Procédé de culture cellulaire comprenant :

   la mise en culture de cellules à 30 à 45 °C dans un milieu dans lequel est dissoute une protéine recombinante de type humain,
   dans lequel la protéine recombinante de type humain est une laminine, un collagène, une gélatine, ou un variant de ceux-ci, et la teneur de la protéine recombinante de type humain dans le milieu est de 0,01 ng/ml à 100 μg/ml,
   dans lequel la protéine recombinante de type humain inclut une gélatine recombinante présentant une séquence d'acides aminés dérivée d'une séquence partielle d'acides aminés de collagène,
   dans lequel la protéine recombinante de type humain présente une séquence répétitive représentée par Gly-X-Y caractéristique du collagène, X et Y représentent chacun indépendamment l'un quelconque d'un acide aminé, une pluralité de morceaux du Gly-X-Y peuvent être identiques ou différents les uns des autres, et la protéine recombinante de type humain inclut deux séquences ou plus de signaux d'adhérence cellulaire dans une molécule,
   dans lequel le signal d'adhérence cellulaire est une séquence d'acides aminés représentée par Arg-Gly-Asp, et dans lequel la séquence d'acides aminés de la protéine recombinante de type humain est représentée par la formule suivante,

   A-[(Gly-X-Y)n]m-B

   dans la formule, A représente n'importe quel acide aminé ou une séquence d'acides aminés, B représente n'importe quel acide aminé ou une séquence d'acides aminés, n morceaux de X représentent chacun indépendamment n'importe quel acide aminé, n morceaux de Y représentent chacun indépendamment n'importe quel acide aminé, et n représente un nombre entier de 3 à 100, m représente un nombre entier de 2 à 10, et n morceaux de Gly-X-Y peuvent être identiques ou différents les uns des autres,
   dans lequel les cellules sont des cellules souches mésenchymateuses,
   et
   dans lequel la protéine recombinante de type humain présente (1) une séquence d'acides aminés décrite dans SEQ ID No : 1, ou (2) une séquence d'acides aminés présentant 80 % ou plus d'identité de séquence avec la séquence d'acides aminés décrite dans SEQ ID No : 1 et présente une adhésivité cellulaire.

2. Procédé de culture cellulaire selon la revendication 1,
   dans lequel le poids moléculaire de la protéine recombinante de type humain est de 2 kDa à 100 kDa.

3. Procédé de culture cellulaire selon l'une quelconque des revendications 1 ou 2,
   dans lequel le poids moléculaire de la protéine recombinante de type humain est de 10 kDa à 90 kDa.

4. Procédé de culture cellulaire selon l'une quelconque des revendications 1 à 3,

   dans lequel une séquence d'acides aminés de la protéine recombinante de type humain est représentée par la formule suivante,

   Gly-Ala-Pro-[(Gly-X-Y)$_{63}$]$_3$-Gly

   dans la formule, 63 morceaux de X représentent chacun indépendamment n'importe quel acide aminé, 63 morceaux de Y représentent chacun indépendamment n'importe quel acide aminé, et 63 morceaux de Gly-X-Y peuvent être identiques ou différents les uns des autres.

## FIG. 1

## FIG. 2

## FIG. 3

6 φ DISH

SILICON well

CULTURE BAG

## FIG. 4

| AFTER SEEDING | 0 MINUTES | 120 MINUTES | 180 MINUTES |
|---|---|---|---|
| LEVEL 1 CBE3 ADDITION | | | |
| LEVEL 2 CBE3 NON-ADDITION | | | |

## FIG. 5

## FIG. 6

## FIG. 7

## FIG. 8

FIG. 9

FIG. 10

EP 3 460 050 B1

FIG. 11

FIG. 12

## REFERENCES CITED IN THE DESCRIPTION

### Patent documents cited in the description

- WO 2017117333 A1
- JP 2010519252 A
- JP 2011078326 A
- WO 2011108517 A
- WO 20091122541 A
- JP 2012175962 A
- WO 2009122541 A
- EP 1014176 A
- US 6992172 B
- WO 200485473 A
- WO 2008103041 A
- EP 1014176 A2
- WO 20081103041 A
- EP 0138092 A2
- JP 17018657 W

### Non-patent literature cited in the description

- **ADAY ; SEZIN ; NESRIN HASIRCI ; ISMET DE-LILOGLU GURHAN.** A cost-effective and simple culture method for primary hepatocytes. *Animal cells and systems,* 2011, vol. 15.1, 19-27
- **ZHAOLIE, CHEN et al.** A novel serum-free medium for the cultivation of vero cells on microcarriers. *Biotechnology techniques,* 1996, vol. 10.6, 449-452
- **HALL, PETER E. ; JUSTIN D. LATHIA ; MAEVE A. CALDWELL.** Laminin enhances the growth of human neural stem cells in defined culture media. *BMC neuroscience,* 2008, vol. 9.1, 71
- **TAKASU, NOBUYUKI et al.** Effect of gelatin on the cyclic AMP response of primocultured hog thyroid cells to acute thyrotropin stimulation. *Biochimica et Biophysica Acta (BBA)-General Subjects,* 1979, vol. 587.4, 507-514
- **JAMBOU, RONAN et al.** In vitro culture of Plasmodium berghei-ANKA maintains infectivity of mouse erythrocytes inducing cerebral malaria. *Malaria Journal,* 2011, vol. 10.1, 346
- Pathophysiology. Nagai Shoten Co., Ltd, 1990, vol. 9, 527
- **MAXEY, C. R.** Phitogr. Gelatin. P. J. Academic, 1976, vol. 2
- **BASIC LOCAL ALIGNMENT SEARCH TOOL (BLAST.** *J. Mol. Biol.,* 1990, vol. 215, 403-410
- *Pharmaceutical Bulletin,* 1954, vol. 2 (2), 163-173
- *Area of Chemistry,* 1957, vol. 11 (10), 719-725
- *Fragrance Journal,* 1981, vol. 50, 79-82
- **YOSHIO KOUDA.** New Edition Organic Conceptual Diagram -Foundation and Application. Sankyo Shuppan Co., Ltd, 2008
- **GASTEIGER E. ; HOOGLAND C. ; GATTIKER A. ; DUVAUD S. ; WILKINS M.R. ; APPEL R.D. ; BAIROCH A.** *Protein Identification and Analysis Tools on the ExPASy Server*
- The Proteomics Protocols. Handbook, Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res,* 2003, vol. 31, 3784-3788
- **NASU ; TAKAYAMA S ; UMEZAWA A.** Endochondral ossification model system: designed cell fate of human-type epiphyseal chondrocytes during long-term implantation. *J. Cell Physiol.,* 2015, vol. 230, 1376-1388